# FASCICULE DE BREVET EUROPEEN

(11) **EP 0 455 561 B1**
(45) Date de publication et mention de la délivrance du brevet: **19.04.1995**
(21) Numéro de dépôt: 91401187.9
(22) Date de dépôt: 06.05.1991
(51) Int. Cl.: A61K 31/74, A61K 9/20

(54) **Nouvelles compositions pharmaceutiques à base de polycarbophil et leur procédé d'obtention**
Neue Polycarbophil enthaltende pharmazeutische Zusammensetzungen und Verfahren zu deren Herstellung
New pharmaceutical compositions containing polycarbophil

(30) Priorité: 04.05.1990 FR 9005651
(43) Date de publication de la demande: 06.11.1991
(73) Titulaire: LABORATOIRE THERAMEX, 98000 Monaco (MC)
(72) Inventeur: Cros, Catherine, Léger 06300 Nice (FR); Languetin, Michel, 06340 La Trinité (FR)
(74) Mandataire: Burtin, Jean-François

(56) Documents cités:
- EP-A- 0 176 773
- EP-A- 0 273 209
- DE-A- 1 811 809
- FR-A- 2 285 856

## Description

La présente invention a pour objet de nouvelles compositions à base de polycarbophil, notamment destinées à l'administration orale, et à leur procédé d'obtention.

Elle a plus particulièrement pour objet un procédé d'obtention de formes sèches à base de polycarbophil qui consiste en ce que l'on mélange ce principe actif avec un agent liant de compression et avec de la polyvinylpyrrolidone puis soumet ce mélange à une compression directe pour former des comprimés que l'on peut protéger en surface, si désiré, par pelliculage, laquage ou enrobage.

Les comprimés ainsi obtenus constituent une forme d'administration particulièrement avantageuse du polycarbophil. En effet, il se pose avec ce principe actif le problème qui s'est déjà posé pour d'autres principes actifs insolubles dans l'eau ou dans un milieux aqueux et qui doivent être administrés à des doses de plusieurs grammes. Il faut donc envisager des présentations en sachets, en poudres ou en granulés qui sont souvent difficiles à ingérer (par ex. le Psyllium) ou de saveur amère difficile à dissimuler ou bien encore, de structure granuleuse (comme la cholestyramine) dont l'absorption est difficile.

Les tablettes à mâcher sont une première approche du problème. Elles résolvent peut-être le probème de la sapidité. Elles ne résolvent pas les problème's posés par la structure granuleuse ou collante de la préparation pulvérulente.

De même, les gros comprimés ou les grosses gélules sont souvent difficiles à déglutir et restent collés au palais, malgré l'absorption de liquides.

Le polycarbophil est un principe actif connu depuis 1957 dans le traitement des troubles du tube digestif notamment comme laxatif de lest (bulky agent). Sa dénomination exacte est polycarbophil sel de calcium. C'est le sel de calcium d'un co-polymère lâche d'acide acrylique avec le divinylglycol. Il constitue une structure polymérique lâche (loose) comportant des canaux. Il peut être représenté par la formule suivante : 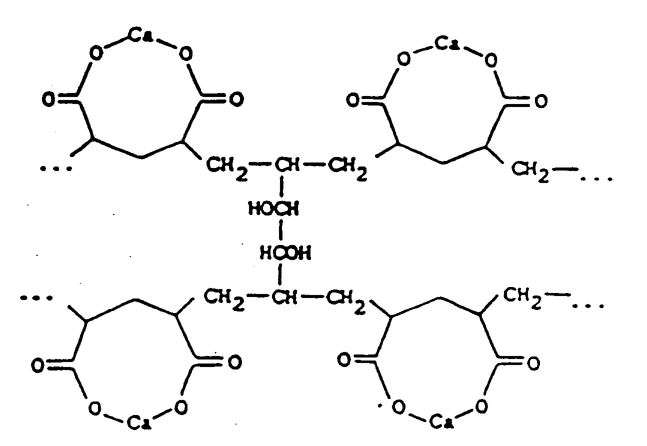
D'après les informations fournies par le fabriquant, le co-polymère acide est neutralisé partiellement (environ 82%) par du calcium et le sel formé contient une proportion certaine d'eau (10%). Il a la propriété d'absorber beaucoup d'eau (à peu près 35 fois et plus son poids d'eau). Son emploi en tant qu'agent anti-diarrhéique et contre la constipation est décrit dans le brevet américain 3.297.664 ainsi que dans la littérature (cf. RUTHLEDGE M.L et al - Curr. Therap. Res. 23 (1978) 443). Il est commercialisé sous deux présentations par la firme GOODRICH - le Carbopol C.977^{R} : de fine granulométrie et le Carbopol EX.83^{R} dont la granulométrie est plus élevée.

La demande de brevet européen 0.273.209 (American Cyanamid) a déjà décrit un mode de réalisation de comprimés après granulation en milieu humide. La masse humide obtenue est broyée et tamisée. Cette poudre après addition d'excipient et de diluants est comprimée puis polliculée.

Cette technique de préparation de comprimés requiert donc un volume important de matériaux inertes et, de ce fait, les comprimés en résultant atteignent une taille et un poids très important (près d'1 g) qui rendent leur ingestion hypothétique.

Il existait donc un réel besoin de trouver une nouvelle formulation qui permette d'administrer le polycarbophil à une dose importante correspondant aux besoins de la thérapeutique, et en même temps, dont le volume reste relativement restreint pour que l'ingestion ne devienne pas un exercice difficile et même parfois, périlleux.

C'est ce que réalise la production de comprimés par compression directe. Elle permet de réduire au maximum la proportion d'agents de dilution, d'agents liants, d'agents de désintégration et d'agents de glissement. De ce fait, le poids total du comprimé sera compris entre 660 et 820 mg et de préférence entre 750 et 810 mg, se qui est tout à fait acceptable pour une ingestion facile. On peut la rendre plus aisée, en réalisent des comprimés de forme piriforme ou losangique et en les recouvrant d'une pellicule protectrice pour en faciliter le glissement.

Les comprimés selon l'invention renferment, en outre, des agents de dilution de la masse du type polysaccharidique comme le lactose, le Néosorb 20/60 (marque déposée au nom de la Société ROQUETTE Frères), le Tablettose (marque déposée au nom de la Société MEGGLE MILCH Industrie GMBH and Co.), le mannitol, le sorbitol ou le mélange lactose/PVP vendu sous la marque Ludipress (Société BASF).

Les agents liants de compression sont en général des celluloses microcristallines comme celles vendues sous la marque AVICEL pH 101 ou AVICEL pH 102 (American Viscose Corporation).

La polyvinylpyrrolidone joue également un rôle important en facilitant l'agglomération des poudres et la compressibilité de la masse. On utilise à cette fin, des polyvinylpyrrolidones de poids moléculaire compris entre 10 000 et 30 000 comme la Povidone, la Kollidon K15 ou la Plasdone XL (Kollidon est une maque déposée au nom de la Société BASF - Plasdone est une marque déposée au nom de la Société GENERAL ANILINE and FILM Corporation).

Le mélange contient également des agents de glissement qui évitent que la poudre soit trop fortement compactée après compression et ne se désintègre plus. On peut citer, à cet égard, les silices colloïdales vendues sous La marque AEROSIL 100 ou AEROSIL 200 (marque déposée au nom de DEGUSSA AG).

Le mélange contient aussi des agents de désintégration qui permettent une désintégration ou un délitement conforme aux normes pharmaceutiques. On pourra citer comme agents de désintégration utiles, les polymères de vinylpyrrolidones réticulées telles que celles vendues sous les marque Polyplasdone ou Polyclar AT (GENERAL ANILINE and FILM Corporation), les carboxyméthylamidons comme ceux vendus sous les marques Amijel (marque déposée par DEUTSCHE MAIZENAWERKE GMBH) ou Explotab (marque déposée par SUOMEN SOKERI -Finlande-), les carboxyméthylcelluloses réticulées ou croscarmelloses comme le composé vendu sous la marque AC-DI-SOL (FMC Corporation).

En outre, la préparation contient des agents de lubrification qui facilitent la compression et le rejet des comprimés. On pourra citer comme agents de lubrification, le palmitostéarate de glycérol vendu sous la maque Précirol (GATTEFOSSE SFPA), le stéarate de magnésium, l'acide stéarique ou le Talc.
La poudre avant compression pourra, en outre, être additionnée d'agents d'aromatisation comme des arômes naturels en poudre dilués dans du sucre ou absorbés sur silice ou des agents colorants.

La compression est effectuée sur une machine rotative avec des poinçons appropriés.
- le diamètre des comprimés est de 12 à 13 mm. La charge de rupture varie de 5 à 15 kg/cm².

Après compression, les comprimés peuvent être enrobés pour assurer leur conservation ou faciliter leur déglutition.

Les agents d'enrobage sont soit cellulosiques comme le phtalate de cellulose (Sepifilm, Pharmacoat), soit polyvinyliques du type sépifilm ECL, soit saccharosiques comme le sucre pour dragéification du type Sépisperse DR, AS, AP ou K (colorés) (Sépifilm et Sépisperse sont des marques déposées au nom de la Société SEPPIC - Pharmacoat est une marque déposée au nom de la Société SHIN-ETSU CHEMICAL Co.).

Les comprimés enrobés ou non, peuvent, en outre, être colorés en surface ou dans la masse par des colorants végétaux ou synthétiques (par ex. laque au jaune de quinoléine ou E 104).

Les proportions des différents constituants varient selon la nature du comprimé à réaliser. La teneur en polycarbophil (sel de calcium) peut varier de 550 à 640 mg. Les agents de dilution varient de 1 à 10% de la masse totale, les agents de glissement de 0,1 à 2% de la masse totale, les agents liants de compression varient de 2 à 10%, la polyvinylpyrrolidone de 0,5 à 1,5%, les agents de désintégration varient de 2,5 à 5,5% pour la polyvinylpyrrolidone réticulée ou pour le carboxyméthylamidon, de 2,0 à 3,0% pour la croscarmellose.

Les quantités d'agents de lubrification varient en fonction de la nature de l'agent de 0,1 à 2,5%.

La quantité d'agent aromatisant reste très faible. Selon l'arôme choisi, elle varie de 0 à 1%. La quantité d'agent de coloration pourra varier selon l'agent, de 0 à 0,075%.

Les comprimés obtenus selon le procédé de l'invention sont utilisés en thérapeutique pour le traitement de la constipation ou de la diarrhée à des doses qui s'échelonnent de 1 g (enfants de 0 à 6 ans), à 2 g (enfants de 6 à 12 ans) et 4 g chez l'adolescent et l'adulte. Le nombre de comprimés à prendre s'échelonnera donc de 2 à 8 par jour.

L'exemple de réalisation suivant illustre l'invention.

Comprimés à 625 g de Polycarbophil (sel de calcium), soit 500 mg de polycarbophil

| | |
|---|---|
| Polycarbophil (vendu sous la marque Carbopol EX-83 de la Société BF GOODRICH Chemical Company) | 625 g |
| Cellulose microcristalline (commercialisée sour la marque AVICEL pH 102 de la Société AMERICAN VISCOSE Co.) | 40,8 g |
| Lactose | 62 g |
| Polyvinylpyrrolidone réticulée (commercialisée sous la marque Polyplasdone XL de la société GENERAL ANILINE and Film Corporation) | 41,6 g |
| Polyvinylpyrrolidone | 6,8 g |
| Silice colloïdale | 1,2 g |
| Palmitostéarate de glycérol | 3,6 g |
| Stéarate de magnésium | 1,6 g |
| Acide stéarique | 12,8 g |
| Colorant E.104 | 0,6 g |
| Arôme citron en poudre | 4,0 g |
| pour 1 000 comprimés terminés au poids moyen de | 0,800 g |

## Revendications (Revendications pour l'(les) Etat(s) contractant(s) suivant(s): AT, BE, CH, DE, DK, FR, GB, IT, LI, LU, NL, SE)

1. Un procédé d'obtention de nouvelles formes sèches à base de polycarbophil qui consiste a ce que l'on mélange le polycarbophil avec un agent liant de compression et de la polyvinylpyrrolidone réticulée et que l'on soumet ce mélange à une compression directe pour former des comprimés que l'a peut protéger en surface par pelliculage, laquage ou enrobage.

2. Un procédé selon la revendication dans lequel le mélange avant compression est additionné d'agents de dilution du type polysaccharidique.

3. Un procédé selon la revendication 1 et la revendication 2 dans lequel, le mélange avant compression directe est additionné en outre d'agents de glissement.

4. Un procédé selon l'une des revendications 1 à 3 dans lequel le mélange avant compression est additionné, en outre, d'un agent de lubrification choisi dans le groupe constitué par le palmitostéarate de glycéryle, le stéarate de magnésium, l'acide stéarique et le talc.

5. Un procédé selon l'une des revendications 1 à 4 dans lequel la teneur en polycarbophil (sel de calcium) varie de 550 à 650 mg.

6. Un procédé selon l'une des revendications 1 à 5 dans lequel la teneur en agent liant de compression varie de 2 à 10 % par rapport à la masse totale.

7. Un procédé selon l'une des revendications 1 à 6 dans lequel la teneur en agent de dilution varie de 1 à 10%.

8. Un procédé selon l'une des revendications 1 à 7 dans lequel la teneur en agent de désintégration du type polyvinyl pyrrolidone réticulée varie de 2,5 à 5,5%.

9. Un procédé sels l'une des revendications 1 à 8 dans lequel la teneur en polyvinylpyrrolidone varie de 0,5 à 1,5 %.

10. Un procédé selon l'une des revendications 1 à 9° dans lequel la teneur en agent de glissement varie de 0,1 à 2 %.

11. Les nouvelles formes pharmaceutiques sèches obtenues selon le procédé tel que défini dans les revendications 1 à 10°.

12. Une nouvelle forme pharmaceutique sèche selon la revendication 11° à savoir les comprimés obtenus par compression directe de polycarbophil (sel de calcium) renfermant 0,625 g de principe actif par prise unitaire.

## Revendications (Revendications pour l'(les) Etat(s) contractant(s) suivant(s): ES, GR)

1. Un procédé d'obtention de nouvelles formes sèches à base de polycarbophil qui consiste a ce que l'on mélange le polycarbophil avec un agent liant de compression et de la polyvinylpyrrolidone réticulée et que l'on soumet ce mélange à une compression directe pour former des comprimés que l'on peut protéger en surface par pelliculage, laquage ou enrobage.

2. Un procédé selon la revendication dans lequel le mélange avant compression est additionné d'agents de dilution du type polysaccharidique.

3. Un procédé selon la revendication 1 et la revendication 2 dans lequel, le mélange avant compression directe est additionné en outre d'agents de glissement.

4. Un procédé selon l'une des revendications 1 à 3 dans lequel le mélange avant compression est additionné, en outre, d'un agent de lubrification choisi dans le groupe constitué par le palmitostéarate de glycéryle, le stéarate de Magnésium, l' acide stéarique et le talc.

5. Un procédé selon l'une des revendications 1 à 4 dans lequel la teneur en polycarbophil ( sel de calcium) varie de 550 à 650 mg.

6. Un procédé selon l'une des revendications 1 à 5 dans lequel la teneur en agent liant de compression varie de 2 à 10 % par rapport à la masse totale.

7. Un procédé selon l'une des revendications 1 à 6 dans lequel la teneur en agent de dilution varie de 1 à 10%.

8. Un procédé selon l'une des revendications 1 à 7 dans lequel la teneur en agent de désintégration du type polyvinyl pyrrolidone réticulée varie de 2,5 à 5,5%.

9. Un procédé selon l'une des revendications 1 à 8 dans lequel la teneur en polyvinylpyrrolidone varie de 0,5 à 1,5 %.

10. Un procédé selon l'une des revendications 1 à 9° dans lequel la teneur en agent de glissement varie de 0,1 à 2 %.

## Claims (Claims for the following Contracting State(s): AT, BE, CH, DE, DK, FR, GB, IT, LI, LU, NL, SE)

1. A process for producing novel dry formulation based on polycarbophil which consists in that polycarbophil is admixed with a binding agent for tabletting and to cross-linked polyvinylpyrrolidone, and in that this mixture is submitted to direct compression to form tablets which may be superficially protected by film formation, lacking or coating.

2. A process according to claim 1 wherein the mixture before tabletting is added to diluting agents of the polysaccharidic type.

3. A process according to claim 1 and claim 2 wherein the mixture before direct compressing is further added to a glidding agent.

4. A process according to any of claims 1 to 3 wherein the mixture before compression is further added to a lubricating agent selected from the group consisting of glyceryl palmitostearate, magnesium stearate, stearic acied and talcum.

5. A process according to any of claims 1 to 4 wherein the content in polycarbophil as the calcium salt ranges from 550 to 650 mg.

6. A process according to any of claims 1 to 5 wherein the content of binding agent for tabletting ranges from 2 to 10 % reported to the whole mass.

7. A process according to any of claims 1 to 6 wherein the content of diluting agent ranges from 1 to 10 %.

8. A process according to any of claims 1 to 7 wherein the content of disintegrating agent of the cross-linked polyvinylpyrrolidone type ranges from 2.5 to 5.5 %.

9. A process according to any of claims 1 to 8 wherein the content of polyvinylpyrrolidone derivative ranges from 0.5 to 1.5 %.

10. A process according to any of claims 1 to 9 wherein the content of the glidding agent ranges from 0.1 to 2 %.

11. The novel pharmaceutical formulations obtained by the process as defined in claims 1 to 10.

12. The novel dry pharmaceutical formulation according to claim 11 i.e. tablets obtained by direct compression of polycarbophil as the calcium salt, containing 625 g of active ingredient per unit dosage.

## Claims (Claims for the following Contracting State(s): ES, GR)

1. A process for producing novel dry formulation based on polycarbophil which consists in that polycarbophil is admixed with a binding agent for tabletting and to cross-linked polyvinylpyrrolidone, and in that this mixture is submitted to direct compression to form tablets which may be superficially protected by film formation, lacking or coating.

2. A process according to claim 1 wherein the mixture before tabletting is added to diluting agents of the polysaccharidic type.

3. A process according to claim 1 and claim 2 wherein the mixture before direct compressing is further added to a glidding agent.

4. A process according to any of claims 1 to 3 wherein the mixture before compression is further added to a lubricating agent selected from the group consisting of glyceryl palmitostearate, magnesium stearate, stearic acied and talcum.

5. A process according to any of claims 1 to 4 wherein the content in polycarbophil as the calcium salt ranges from 550 to 650 mg.

6. A process according to any of claims 1 to 5 wherein the content of binding agent for tabletting ranges from 2 to 10 % reported to the whole mass.

7. A process according to any of claims 1 to 6 wherein the content of diluting agent ranges from 1 to 10 %.

8. A process according to any of claims 1 to 7 wherein the content of disintegrating agent of the cross-linked polyvinylpyrrolidone type ranges from 2.5 to 5.5 %.

9. A process according to any of claims 1 to 8 wherein the content of polyvinylpyrrolidone derivative ranges from 0.5 to 1.5 %.

10. A process according to any of claims 1 to 9 wherein the content of the glidding agent ranges from 0.1 to 2 %.

## Patentansprüche (Patentansprüche für folgende(n) Vertragsstaat(en): AT, BE, CH, DE, DK, FR, GB, IT, LI, LU, NL, SE)

1. Verfahren fur Herstellung neuen trockenen Formulierungen auf Basis von Polycarbophil dadurch gekennzeichnet dass Polycarbophil mit einer Bindungsmittel für Verpressing und mit vernetzten Polyvinylpyrrolidon vermischt wird und dass diese Mischung einer direkt verpressung unterworfen wird um Tabletten zugewinnen die oterflächig durch Film bildung, Lackierung oder Umhüllung schützen kann.

2. Verfahren nach Anspruch 1 worin die Mischung vor der Verpressung mit Verdünnungs mitteln des Polysaccharids-Art zugesetzt wird.

3. Verfahren nach Anspruch 1 und Anspruch 2 worin die Mischung vor der direkten Verpressung desweiteren mit Glattmitteln zugesetzt wird.

4. Verfahren nach Ansprüche 1 bis 3, worin die Mischung vor Verpressung desweiteren mit einem Gleitmittel aus der Gruppe vor Glyzeryl palmitostearat, Magnesium Stearat, Stearsäure, und Talkum gebildet ist.

5. Verfahren nach einer der Ansprüche 1 bis 4, worin der Gehalt an Polycarbophil als Calzium Salz, von 550 bis 650 mg variiert.

6. Verfahren nach einer der Ansprüche 1 bis 5, worin der Gehalt an Bindungsmittel fur Verpressung, von 2 bis 10 % bezogen auf die gesamte Masse, variiert.

7. Verfahren nach einer der Ansprüche 1 bis 6, worin der Gehalt an Verdünnungsmittel von 1 bis 10 % variiert.

8. Verfahren nach einer der Ansprüche 1 bis 7, worin der Gehalt an Zerfallungsmittel des vernetzen Polyvinylpyrrolidon Art von 2.5 bis 5.5 % variiert.

9. Verfahren nach einer der Ansprüche 1 bis 8, worin der Gehalt an Polyvinylpyrrolidon von 0.5 bis 1.5 % variiert.

10. Verfahren nach einer der Ansprüche 1 bis 9, worin der Gehalt an Gleitmittel von à. 1 bis 2 % variiert.

11. Die neue trockene pharmazeutische Formulierungen die nach dem Verfahren als in der Ansprüche 1 bis 10 geschreibt, hergestellt werden.

12. Eine neue trockene pharmazeutische Formulierung nach Anspruch 11, d.h. die Tabletten die durch direkte Verpressung von Polycarbophil als Calzium Salz, hergestellt sind und die 0.625 g wirkstoff per einzelne Dosis enthalten.

## Patentansprüche (Patentansprüche für folgende(n) Vertragsstaat(en): ES, GR)

1. Verfahren fur Herstellung neuen trockenen Formulierungen auf Basis von Polycarbophil dadurch gekennzeichnet dass Polycarbophil mit einer Bindungsmittel für Verpressing und mit vernetzten Polyvinylpyrrolidon vermischt wird und dass diese Mischung einer direkt verpressung unterworfen wird um Tabletten zugewinnen die oterflächig durch Film bildung, Lackierung oder Umhüllung schützen kann.

2. Verfahren nach Anspruch 1 worin die Mischung vor der Verpressung mit Verdünnungsmitteln des Polysaccharids-Art zugesetzt wird.

3. Verfahren nach Anspruch 1 und Anspruch 2 worin die Mischung vor der direkten Verpressung desweiteren mit Glattmitteln zugesetst wird.

4. Verfahren nach Ansprüche 1 bis 3, worin die Mischung vor Verpressung desweiteren mit einem Gleitmittel aus der Gruppe vor Glyzeryl palmitostearat, Magnesium Stearat, Stearsäure, und Talkum gebildet ist.

5. Verfahren nach einer der Ansprüche 1 bis 4, worin der Gehalt an Polycarbophil als Calzium Salz, von 550 bis 650 mg variiert.

6. Verfahren nach einer der Ansprüche 1 bis 5, worin der Gehalt an Bindungsmittel fur Verpressung, von 2 bis 10 % bezogen auf die gesamte Masse, variiert.

7. Verfahren nach einer der Ansprüche 1 bis 6, worin der Gehalt an Verdünnungsmittel von 1 bis 10 % variiert.

8. Verfahren nach einer der Ansprüche 1 bis 7, worin der Gehalt an Zerfallungsmittel des vernetzen Polyvinylpyrrolidon Art von 2.5 bis 5.5 % variiert.

9. Verfahren nach einer der Ansprüche 1 bis 8, worin der Gehalt an Polyvinylpyrrolidon von 0.5 bis 1.5 % variiert.

10. Verfahren nach einer der Ansprüche 1 bis 9, worin der Gehalt an Gleitmittel von à. 1 bis 2 % variiert.
